Europäisches Patentamt

European Patent Office

Office européen des brevets

(19)

(11) **EP 1 086 067 B1**

(12) **EUROPÄISCHE PATENTSCHRIFT**

(45) Veröffentlichungstag und Bekanntmachung des
Hinweises auf die Patenterteilung:
**03.09.2003 Patentblatt 2003/36**

(51) Int Cl.$^7$: **C07C 29/70**

(21) Anmeldenummer: **99927790.8**

(86) Internationale Anmeldenummer:
**PCT/EP99/03713**

(22) Anmeldetag: **28.05.1999**

(87) Internationale Veröffentlichungsnummer:
**WO 99/065849 (23.12.1999 Gazette 1999/51)**

(54) **VERFAHREN ZUR HERSTELLUNG VON ALKALIMETALLALKOHOLATEN**

METHOD FOR PRODUCING ALKALI METAL ALCOHOLATES

PROCEDE DE PRODUCTION D'ALCOOLATS DE METAUX ALCALINS

(84) Benannte Vertragsstaaten:
**AT BE CH CY DE DK ES FI FR GB GR IE IT LI LU
MC NL PT SE**

(30) Priorität: **12.06.1998 DE 19826394
05.05.1999 DE 19920594**

(43) Veröffentlichungstag der Anmeldung:
**28.03.2001 Patentblatt 2001/13**

(73) Patentinhaber: **BASF AKTIENGESELLSCHAFT
67056 Ludwigshafen (DE)**

(72) Erfinder:
 • **FRIEDRICH, Holger
  D-67240 Bobenheim-Roxheim (DE)**
 • **GUTH, Josef
  D-67251 Freinsheim (DE)**
 • **SCHWEINZER, Jürgen
  D-67227 Frankenthal (DE)**

 • **LETZELTER, Thomas
  D-76855 Anweiler (DE)**
 • **BENDER, Hans-Jürgen
  D-67251 Freinsheim (DE)**

(74) Vertreter: **Isenbruck, Günter, Dr. et al
Isenbruck, Bösl, Hörschler, Wichmann, Huhn,
Patentanwälte
Theodor-Heuss-Anlage 12
68165 Mannheim (DE)**

(56) Entgegenhaltungen:
 EP-A- 0 810 193          DE-B- 1 251 297
 DE-C- 973 323           FR-A- 850 126
 US-A- 5 262 133          US-A- 5 583 269

 • CHEMICAL ABSTRACTS, vol. 86, no. 11, 14. März
  1977 (1977-03-14) Columbus, Ohio, US; abstract
  no. 71899m, Seite 551; XP002113639 & CS 163
  902 A (J. VACEK, ET AL.) 15. Juli 1976
  (1976-07-15)

EP 1 086 067 B1

**Beschreibung**

Die Erfindung betrifft ein Verfahren zur Herstellung von Alkalimetallalkoholaten, insbesondere von tertiären Alkoholaten wie Natrium-tert.-Butanolat.

Die Herstellung von Alkalimetallalkoholaten (auch Alkalimetallalkoxide genannt) durch Umsetzung von Alkalimetallen mit Alkoholen ist allgemein bekannt. Die Kettenlänge und die Struktur des Alkohols haben bei dieser Umsetzung einen entscheidenden Einfluß auf die Reaktionsgeschwindigkeit. Die Reaktionsgeschwindigkeit nimmt mit zunehmender Kettenlänge und zunehmender Verzweigung der Alkohole ab. Primäre Alkohole reagieren sehr schnell, tertiäre Alkohole hingegen äußerst träge. Die Umsetzung von tertiären Alkoholen dauert in der Regel mehrere Stunden bis mehrere Tage.

Weiterhin wird während der Reaktion häufig die Sättigungskonzentration des jeweiligen Alkoholats im Alkohol überschritten. Das Produkt fällt aus und verkrustet das Alkalimetall, wodurch dieses an der Weiterreaktion gehindert wird. Dies ist insbesondere dann der Fall, wenn die Temperatur nicht hoch genug ist, um das Alkalimetall aufzuschmelzen und durch Rühren fein zu dispergieren.

Die geringe Reaktionsgeschwindigkeit bereitet Probleme bei der Reaktionsdurchführung. Es können nur geringe Raumzeitausbeuten erzielt werden, was letztendlich zu hohen Kosten führt.

Je nach Herstellungsbedingungen werden bei der Alkoholatherstellung pro Mol Alkoholat bis zu 3 Mol Alkohol als Kristallalkohol gebunden. Mitunter kristallisieren diese Alkohol-Alkoholatkomplexe zuerst aus, wenn die Umsetzung in einem inerten Lösungsmittel durchgeführt wird. Die herstellungsbedingte Kristallalkoholanlagerung führt dazu, daß das Alkoholat in vielen Lösungsmitteln unlöslich wird. Der Kristallalkohol läßt sich nur schwer von den Alkoholaten abspalten, und dies kann in nachfolgenden Reaktionsschritten zu unerwünschten Nebenreaktionen führen. Ziel ist daher die Herstellung kristallalkoholfreier Alkoholate in möglichst kurzen Reaktionszeiten.

Es wurden unterschiedliche Verfahren vorgeschlagen, um Alkalimetallalkoholate, inbesondere kristallalkoholfreie tertiäre Alkalimetallalkoholate in ausreichender Reaktionsgeschwindigkeit herzustellen.

Die DE-A 23 33 634 beschreibt ein Verfahren zur Herstellung kristallalkoholfreier, in indifferenten Lösungsmitteln löslicher Alkali- und Erdalkalimetallalkoholate durch Umsetzung von Alkoholen wie Iso-Alkoholen, Carbinolen, sekundären, tertiären aber auch primären Alkoholen mit Alkali- oder Erdalkalimetallen in äquimolarer Menge oder im Überschuß. Die Umsetzung erfolgt in einem inerten Lösungsmittel bei erhöhtem Druck und Temperaturen, bei denen sich unter Normaldruck Kristallalkohol abspalten würde. Da das Alkalimetall in diesem Temperatur/Druckbereich in geschmolzener Form vorliegt, läßt es sich gut dispergieren, wodurch eine erhöhte Reaktionsgeschwindigkeit erreicht wird, und kann in größeren Stücken zugegeben werden. Die Reaktionszeiten sind dennoch relativ lang. Der erhöhte Druck erfordert den Einsatz von Druckapparaturen, was mit zusätzlichen Kosten verbunden ist. Der bevorzugt eingesetzte Metallüberschuß führt nach mehreren Reaktionen zur Anreicherung von Verunreinigungen, die die Umsetzung stören können und Reinigungsoperationen erforderlich machen.

Die DE-C 26 12 642 offenbart ein Verfahren zur Herstellung von kristallalkoholfreien, in inerten Lösungsmitteln löslichen Alkalimetallalkoholaten durch Umsetzung von tertiären Alkoholen wie tert.-Butylalkohol und tert.-Amylalkohol mit stöchiometrischen Mengen Alkalimetall. Die Umsetzung wird in einem inerten Lösungsmittel bei einer Temperatur oberhalb des Schmelzpunktes des Alkalimetalls durchgeführt und unterhalb der Abspaltungstemperatur des Kristallalkohols. Das Alkalimetall liegt, zur Erhöhung der Reaktionsgeschwindigkeit, in dispergierter Form (Teilchengröße < 100 μm) vor. Die Umsetzung von z.B. tert.-Butanol muß auch hier unter Druck durchgeführt werden.

In der DD 298 502 wird ein Verfahren zur Herstellung kristallalkoholfreier Alkalimetall-Alkoholate beschrieben, in dem Alkohole, unter anderem tertiäre Alkohole, vorzugsweise in einem geringen Überschuß, mit Alkalimetallen in einem inerten Lösungsmittel umgesetzt werden. Die Umsetzung erfolgt bei einer Temperatur bei der das Alkalimetall geschmolzen ist und der Alkohol dampfförmig vorliegt. Der Alkohol wird kontinuierlich rekondensiert und in die Reaktionsmischung zurückgeführt.

Die JP-A 05 170680 (= DW93/252679) offenbart ein Verfahren zur Herstellung von kristallalkoholfreiem Natrium-tert.-Butanolat durch Umsetzung von tert.-Butylalkohol mit Natrium unter Zugabe von 5 bis 15 Gew.-% eines inerten Lösungsmittels bei einer Temperatur von 130 bis 135°C. Dabei wird das geschmolzene Natrium in die Reaktionsmischung eingesprüht. Überschüssiger Alkohol und Lösungsmittel werden abdestilliert. Dieses Verfahren ist sehr aufwendig. Zudem können die benötigten Düsen leicht verstopfen, so daß das Verfahren störanfällig ist.

In der EP-A 0 749 947 ist ein kontinuierliches Verfahren zur Herstellung von $C_4$- bis $C_8$-Natriumalkoholaten offenbart. In diesem Verfahren wird eine Dispersion von Natrium in einem inerten Lösungsmittel mit der 0,4 bis 0,6 molaren Menge Alkohol bei erhöhter Temperatur (100 bis 140°C) umgesetzt. Von der entstandenen zweiphasigen Reaktionsmischung wird die obere, inertes Lösungsmittel und Alkoholat enthaltende Phase abdekantiert und die Untere, überschüssiges Natrium enthaltende Phase mit frischem Natrium versetzt und erneut umgesetzt. Ein Nachteil dieses Verfahrens ist, daß das Reaktionsprodukt umständlich abdekantiert werden muß.

Alle oben genannten Verfahren benötigen zusätzliches hochsiedendes, inertes Lösungsmittel. Die Entfernung des Lösungsmittels ist in der Regel aufwendig und zudem meist unvollständig. Diese zusätzlichen Verunreinigungen können z.B. bei der Verwendung der Alkoholate in Pharmasynthesen stören. Zudem werden durch den Einsatz eines zusätzlichen Lösungsmittels weitere Kosten für die Reinigung des Lösungsmittels, für Lagertanks und Versorgungsleitungen etc. verursacht. Die erhaltenen Alkohole werden üblicherweise direkt als Lösungen in den entsprechenden inerten Lösungsmitteln weiterverwendet. Zur Gewinnung von festen Alkoholaten ist es erforderlich, die Lösungen in z. B. einen Trockner zu überführen und vom Lösungsmittel zu befreien.

US 5,583,269 betrifft ein Verfahren zur Herstellung von Lösungen tertiärer Lithiumalkoholate. Die Umsetzung wird mit einem Überschuß Lithium durchgeführt, wobei das Lithium in Lithiumstücken vorliegt, die weniger als 0,1 Gew.-% Natrium enthalten. Die Umsetzung wird mit Ether und/oder Kohlenwasserstoffen als Lösungsmittel durchgeführt. Als Katalysator können geringe Mengen von $C_1$- bis $C_3$-Alkoholen zugegeben werden.

Die EP-A 0 192 608 beschreibt ein Verfahren zur Herstellung von Alkalimetallalkoholaten tertiärer Alkohole durch Umsetzung eines Alkalimetalls mit einem tertiären Alkohol ohne Zugabe inerter Lösungsmittel. Dabei wird der heiße Alkohol unter Rühren zum geschmolzenen Alkalimetall gegeben. Der Alkohol wird in einem 3 bis 6, vorzugsweise 5 molaren Überschuß eingesetzt. Dieses Verfahren hat den Nachteil, daß Alkohole mit Siedepunkten unterhalb des Schmelzpunktes des eingesetzten Alkalimetalls nicht mit ausreichender Reaktionsgeschwindigkeit umgesetzt werden können, da das feste Alkalimetall im Lösungsmittel nicht ausreichend dispergiert werden kann. Die erhaltenen Alkalimetallalkoholate werden direkt in alkoholischer Lösung weiterverwendet.

Es bestand daher die Aufgabe, ein Verfahren zur Herstellung von Alkalimetallalkoholaten bereitzustellen, das keine zusätzlichen hochsiedenden Lösungsmittel benötigt und bei dem Alkohole, insbesondere reaktionsträge Alkohole mit Siedepunkten unterhalb des Schmelzpunktes des eingesetzten Alkalimetalls, wie tert.-Butanol, vollständig mit Alkalimetallen umgesetzt werden können. Die Umsetzung soll möglichst schnell und bei Normaldruck verlaufen und die Alkalimetallalkoholate sollen kristallalkoholfrei gewonnen werden. Des weiteren soll die Gewinnung der Alkalimetallalkoholate in fester Form ohne hohen apparativen Aufwand ermöglicht werden.

Diese Aufgabe wird durch ein Verfahren zur Herstellung von Alkalimetallalkoholaten durch Umsetzung von Alkalimetallen mit tertiären Alkoholen gelöst, wobei die Umsetzung in Gegenwart eines Übergangsmetallsalzes als Katalysator erfolgt.

Das erfindungsgemäße Verfahren ermöglicht eine schnelle und vollständige Umsetzung auch von reaktionsträgen tertiären Alkoholen unterhalb der Schmelztemperatur des jeweiligen Alkalimetalls bei Normaldruck. Die Verwendung hochsiedender Lösungsmittel und teurer Druckapparaturen ist nicht notwendig. Dadurch wird ein kostengünstiges, einfach durchzuführendes und wirtschaftliches Verfahren bereitgestellt.

Als Katalysatoren werden Übergangsmetallsalze, vorzugsweise in Form ihrer alkoholischen Lösungen, eingesetzt.

Als Übergangsmetallsalze eignen sich besonders Salze von Metallen der 4. bis 8. Nebengruppe des Periodensystems der Elemente. Besonders bevorzugt werden Salze des Fe, Ni, Cr, Ti, Co, Pt, Pd, V, Nb, W, Mo oder Mn sowie des Rh oder Ru eingesetzt. Ganz besonders bevorzugt ist der Einsatz von Eisensalzen.

Des weiteren können Salze des Zn, Cu, Ag oder Au eingesetzt werden.

Geeignete Salze sind beispielsweise Chloride, Bromide, Iodide, Nitrate, Acetate, Formiate, Oxalate und andere Carboxylate, Komplexe von β-Diketonen und β-Ketocarbonsäurederivaten sowie Alkoholate. Bevorzugt werden Chloride eingesetzt.

Als Katalysatoren können beispielsweise Eisen (III)octoat, Titantetrabutylat oder Eisen(III)chlorid eingesetzt werden.

Ganz besonders bevorzugt ist der Einsatz von Eisen(III)chlorid als Katalysator.

Das Übergangsmetallsalz wird vorzugsweise in sehr geringen Mengen, im ppm-Bereich, eingesetzt. Berechnet auf die eingesetzte Menge Alkalimetall wird eine Menge von 1 - 1000 ppm, bevorzugt 10 - 100 ppm, berechnet als Metall, verwendet. Beispielsweise werden bei Einsatz von Eisen(III)chlorid als Katalysator zwischen 30 und 300 mg Eisen(III)chlorid pro kg Natrium eingesetzt.

Die erfindungsgemäße Umsetzung des Alkalimetalls mit dem Alkohol erfolgt bevorzugt ohne zusätzliches Lösungsmittel. Der entsprechende Alkohol wird vorzugsweise im Überschuß eingesetzt und dient gleichzeitig als Dispergiermedium und Lösungsmittel. Die Höhe des Alkoholüberschusses ist im allgemeinen abhängig von der Löslichkeit des Alkoholats im entsprechenden Alkohol. Üblicherweise werden die molaren Verhältnisse von Alkohol zu Alkalimetall so gewählt, daß eine rührbare Suspension des Alkoholats im Alkohol entsteht. Der Alkohol wird im allgemeinen in einem mindestens 2 molaren Überschuß eingesetzt. Bevorzugt ist ein mindestens 5 molarer Überschuß des Alkohols im Verhältnis zum eingesetzten Alkalimetall, besonders bevorzugt ist ein 5 - 10 molarer Überschuß.

Als Alkohole werden tertiäre Alkohole eingesetzt, bevorzugt mit 4 bis 10 C-Atomen, besonders bevorzugt tert.-Butanol und tert.-Amylalkohol. Ganz besonders vorteilhaft ist die Anwendung des erfindungsgemäßen Verfahrens bei reaktionsträgen Alkoholen mit Siedepunkten unterhalb des Schmelzpunktes des eingesetzten Alkalimetalls. tert.-Butanol wird daher ganz besonders bevorzugt eingesetzt. Ein Beispiel ist die Herstellung von Natrium-tert.-Butanolat aus Natrium (Schmelzpunkt 98°C) und tert.-Butanol (Siedepunkt 82°C).

Das Verfahren kommt auch bei niedrigsiedenden, reaktionsträgen Alkoholen ohne zusätzliches hochsiedendes Lösungsmittel oder die Anwendung von Druck aus.

Die Umsetzung des Alkohols mit dem Alkalimetall kann bei einer Temperatur unterhalb des Schmelzpunktes des Alkalimetalls erfolgen. Bevorzugt wird die Umsetzung bei oder in der Nähe der Siedetemperatur des jeweiligen Alkohols durchgeführt. So wird die Umsetzung von tert.-Butanol mit Natrium beispielsweise in siedendem tert.-Butanol durchgeführt, was einer Temperatur von ca. 82°C entspricht.

Als Alkalimetall sind prinzipiell alle Alkalimetalle geeignet. Bevorzugt werden Li, K oder Na eingesetzt, besonders bevorzugt K oder Na, ganz besonders bevorzugt Na.

Darin zeigt sich ein besonderer Vorteil des erfindungsgemäßen Verfahrens. Bei der Umsetzung niedrigsiedender, reaktionsträger Alkohole kann das preiswerte Natrium trotz seines im Vergleich zum teuereren Kalium hohen Schmelzpunktes eingesetzt werden. Dadurch ist das erfindungsgemäße Verfahren sehr kostengünstig.

In einer bevorzugten Ausführungsform wird das Alkalimetall zunächst in flüssiger Form zu einer Mischung aus Alkohol und Katalysator zudosiert. Bei Verwendung von Natrium wird dieses auf ca. 120 - 130°C erwärmt, so daß eine Schmelze entsteht. Diese wird unter Rühren zu der Reaktionsmischung getropft, oder man läßt das geschmolzene Natrium in einem dünnen Strahl zulaufen. Eine besonders feine Verteilung des Alkalimetalls ist in dem erfindungsgemäßen Verfahren nicht erforderlich. Geeignete Partikel haben im allgemeinen mittlere Durchmesser zwischen 1 und 10 mm. Die Umsetzungstemperatur liegt bevorzugt im Bereich der Siedetemperatur des jeweiligen Alkohols.

Das erfindungsgemäße Verfahren eignet sich auch, um tertiäre Alkohole mit einem .Siedepunkt oberhalb der Schmelztemperatur des Alkalimetalls umzusetzen, indem man eine Temperatur oberhalb der Schmelztemperatur wählt. Auch hier bringt der Einsatz des Katalysators eine Verkürzung der Reaktionszeiten und damit eine Verringerung der Chargenzeiten.

So kann z.B. bei der Umsetzung von tert.-Amylalkohol mit Natrium bei der Siedetemperatur des Alkohols (ca. 102°C) gearbeitet werden. Das Natrium ist bei dieser Temperatur flüssig und wird mit dem Rührer dispergiert. Die Reaktionszeit hängt unter diesen Bedingungen von der Rührerdrehzahl und der Art und Konzentration des Katalysators ab. Hohe Drehzahlen führen hier zu einer guten Dispergierung und damit zu einer schnellen Reaktion, die durch Zusatz des erfindungsgemäßen Katalysators weiter gesteigert werden kann.

Die Reaktionsdauer ist unter anderem abhängig von der Ansatzgröße, dem eingesetzten Alkohol und dem eingesetzten Alkalimetall. Die Reaktion wird anhand des freiwerdenden Wasserstoffs verfolgt. Das Ende der Wasserstoffentwicklung zeigt das Ende der Umsetzung des Alkohols mit dem Alkalimetall an.

Die Umsetzung kann in Lösung oder in einer Suspension durchgeführt werden. Sie wird dabei bevorzugt so durchgeführt, daß eine Suspension des Alkoholats in dem entsprechenden Alkohol entsteht. Ein Großteil des Alkoholats liegt dabei auch gelöst vor.

Bei der Herstellung von Natrium-tert.-amylat erhält man in einer bevorzugten Ausführungsform zunächst eine Lösung des Alkoholats im entsprechenden Alkohol. Da die Löslichkeit von Natrium-tert.-amylat im Amylalkohol mit ca. 30 bis 35 Gew.-% bei 100°C recht hoch ist, ist die Umsetzung in einer Suspension hier nicht notwendig.

Die bevorzugte Produktkonzentration des Alkoholats in dem entsprechenden Alkohol ist abhängig von dem eingesetzten Alkohol bzw. der Löslichkeit des entsprechenden Alkoholats in dem Alkohol. Es sind, in Abhängigkeit vom verwendeten Alkohol, Produktkonzentrationen von bis zu 60 Gew.-% Alkoholat in dem entsprechenden Alkohol möglich. Bei der Herstellung von Natrium-tert.-Butanolat ist eine Produktkonzentration von 20 - 30 Gew.-% tert.-Butanolat in tert.-Butanol bevorzugt, besonders bevorzugt sind ca. 15 Gew.-%.

Die hergestellten Alkoholate werden im allgemeinen in fester Form als kristallalkoholfreie Alkoholate gewonnen. Diese sind, in Abhängigkeit von ihrer Struktur und Kettenlänge, in zahlreichen Lösungsmitteln löslich und in einer Vielzahl von Reaktionen einsetzbar.

In einer bevorzugten Ausführungsform werden die Alkalimetallalkoholate im Anschluß an die Umsetzung durch gegebenenfalls Eindampfen von überschüssigem Alkohol und Trocknen in fester, kristallalkoholfreier Form gewonnen.

Das Eindampfen des überschüssigen Alkohols und Trocknen des entstandenen Feststoffs erfolgt dabei im allgemeinen bei einer Temperatur oberhalb des Siedepunktes des verwendeten Alkohols im Anschluß an die Umsetzung.

Die Trocknung der Alkoholate erfolgt, in Abhängigkeit vom verwendeten Alkohol, im allgemeinen in einem Temperaturbereich von 90 - 230°C, bevorzugt 100 - 200°C, besonders bevorzugt 110 - 180°C bei Normaldruck.

Durch Anlegen eines Vakuums im allgemeinen bis maximal 200 mbar, bevorzugt von 10 - 100 mbar, besonders bevorzugt von 10 - 30 mbar, kann die Trocknung beschleunigt und die Trockenwirkung verbessert werden. Des weiteren können bei Verwendung von Vakuum die angegebenen Temperaturbereiche unterschritten werden. Die Temperatur und das Vakuum sind so zu wählen, daß möglichst keine oder nur geringe Verluste durch Sublimation des Alkoholats auftreten.

Besonders bevorzugt werden die Umsetzung, das Eindampfen und das Trocknen in einer einzigen Apparatur durchgeführt.

Die aus dem Stand der Technik bekannten Verfahren zur Herstellung von kristallalkoholfreien Alkalimetallalkoholaten betreffen die Herstellung der Alkalimetallalkoholate in Lösung. Das Lösungsmittel ist darin im allgemeinen ein inertes hochsiedendes Lösungsmittel. Um feste Alkalimetallalkoholate zu erhalten, müssen diese Lösungen in eine separate, zur Eindampfung und Trocknung geeignete Apparatur überführt werden. Der Einsatz mehrerer Apparaturen führt jedoch zu hohen Investitionskosten sowie zu zusätzlichen Arbeitsschritten, die das Produkt verteuern.

Zur Herstellung von festen, kristallalkoholfreien Alkalimetallalkoholaten gemäß des erfindungsgemäßen Verfahrens in einer einzigen Apparatur sind solche Reaktoren geeignet, die die Ablagerung von festem Alkalimetallalkoholat an der Reaktorwand weitgehend verhindern und für eine gute Durchmischung des Reaktionsgemisches geeignet sind. Es werden somit bevorzugt Reaktoren mit einem wandgängigen Rührorgan eingesetzt. Der Rührer-Wand-Abstand beträgt im allgemeinen 0,1 bis 5 cm, bevorzugt 0,1 bis 1 cm, besonders bevorzugt 0,1 bis 0,8 cm.

Besonders bevorzugt werden Reaktoren mit Rührorganen eingesetzt, in denen Froude-Zahlen von > 0,1, bevorzugt von > 0,5, besonders bevorzugt von > 1,0 realisiert werden können.

Die Froude-Zahl stellt ein Maß für die Intensität des Mischens dar und ist definiert als

$$Fr = \frac{(2\pi f)^2 \cdot r}{g},$$

wobei

g    Erdbeschleunigung [9,81 m/s$^2$]

r    Radius [m]

f    Frequenz [1/s]

bedeuten. Die Frequenz wird aus der Drehzahl der Mischwerkzeuge bestimmt. Der Radius ist der größte Abstand zwischen Mischwerkzeug und Welle.

Geeignete Reaktor- bzw. Mischertypen sind beispielsweise ausgewählt aus List-Trockner (z.B Diskotherm-Reaktor), Rührkessel mit Ankerrührer, Schaufeltrockner, Rührkessel mit Wendelrührer, Pflugscharmischer (Lödigemischer) und Eirichmischer.

Im Sinne der vorliegenden Erfindung ist unter einem Schaufeltrockner ein zylindrischer Reaktor, der in der Regel liegend angeordnet ist, zu verstehen. In diesem Reaktor rotieren an einer horizontalen Welle schaufelförmige Paddel (Rührorgan). Der Paddel-Wandabstand ist - mindestens im unteren Bereich - gering, so daß dickere Wandbeläge verhindert werden.

List-Trockner können einwellige (z.B. Diskotherm-Reaktor) und zweiwellige Apparate sein, die sich dadurch auszeichnen, daß sie außer den auf der Hauptwelle befindlichen Rührorganen Gegenhaken an der Wand haben (einwelliger Apparat) bzw. eine zweite Welle mit "Putzhaken" haben. Diese Haken bzw. Putzwellen dienen durch ein intensives ineinandergreifen zum Durchmischen oder Durchkneten des Produkts und zum Abreinigen der produktberührten Flächen, was vor allem bei pastösen oder krustenden Produkten erforderlich ist, um keine Einschränkungen im Wärmeübergang zu bekommen.

Ein Rührkessel mit Ankerrührer kann z.B. einen geschränkten Ankerrührer aufweisen, also ein Rührorgan, welches einen geringen Abstand zur Wand aufweist. Der Ankerrührer sorgt für eine gute Durchmischung eines Ansatzes. Aufgrund der Wandgängigkeit verhindert er, daß dickere Krusten an der Wand anwachsen. Demzufolge ist ein solcher Apparat auch zum Eindampfen und Trocknen geeignet.

Ein Pflugscharmischer (Lödigemischer) ist ein liegender, zylindrischer Reaktor. Um eine horizontale Welle rotieren Mischwerkzeuge, die eine den in der Landwirtschaft verwendeten Pflügen nachempfundene Form haben. Der Abstand des Mischwerkzeugs zur Wand ist gering.

Der Eirichmischer ist ein Mischer, bei dem ein schräg angeordneter Mischtrog rotiert. Wandanbackungen werden durch einen Wandabstreifer verhindert, der hier feststeht, da der Behälter rotiert.

Die entsprechenden Rührorgane werden im allgemeinen mit Drehzahlen von 5 bis 500 Upm, bevorzugt von 5 bis 200 Upm, besonders bevorzugt von 10 bis 100 Upm betrieben.

Bevorzugt enthalten die Reaktoren zusätzlich zu dem eingesetzten Rührorgan ein weiteres Rühr- oder Zerkleinerungsorgan. Dieses wird im allgemeinen, in Abhängigkeit von der Größe der Reaktoren und den eingesetzten Rühr- und Zerkleinerungsorganen, mit Drehzahlen von 500 bis 20000 Upm, bevorzugt von 1000 bis 10000 Upm, besonders bevorzugt von 1000 bis 5000 Upm betrieben. Die mit diesem Rührorgan erzielten Froude-Zahlen liegen üblicherweise bei >1. Das weitere Rühr- oder Zerkleinerungsorgan dient zur Erzeugung hoher Scherfelder, wodurch auch bei Temperaturen unterhalb des Schmelzpunktes des Alkalimetalls ein Zusammenbacken des Alkalimetalls sowie ein Zusammenbacken bzw. Anbacken des gebildeten und gegebenenfalls teilweise aus der Lösung ausgefallenen (in Suspension vorliegenden) Alkalimetallalkoholats an der Reaktorwand verhindert wird. Des weiteren werden Alkalimetallalkoholatkrusten auf dem Alkalimetall entfernt.

Dadurch wird eine besonders hohe Reaktionsgeschwindigkeit der Reaktanden erzielt, so daß nur sehr geringe Mengen an Katalysator erforderlich sind.

Im allgemeinen steigt die Wirksamkeit des Rühr- oder Zerkleinerungsorgans mit steigender Drehzahl. Irgendwann wird jedoch ein Bereich erreicht, wo eine weitere Steigerung keine Vorteile mehr bringt und wo eine weitere Steigerung nicht mehr wirtschaftlich ist.

Zudem muß der Drehzahlbereich so gewählt werden, daß keine Kavitation eintritt (was bei sehr hohen Drehzahlen der Fall sein kann). Optimale Drehzahlen müssen für den jeweils eingesetzten Reaktortyp und die ausgewählte Kombination der Rührorgane in Routineversuchen ermittelt werden.

Als zusätzliche Rühr- oder Zerkleinerungsorgane sind, in Abhängigkeit von den eingesetzten Rühr- bzw. Mischertypen, insbesondere Messermühlen, Zerhacker, Dissolverscheiben, Scheibenrührer, Schrägblattrührer, Turrax-Rührer® bzw. Ultraturrax® oder Wirbler geeignet.

Gemäß der vorliegenden Erfindung sind unter Messermühlen schnell rotierende Messer, die für die Zerkleinerung von Feststoffen sorgen, zu verstehen.

Dissolverscheiben sind schnellrotierende Scheiben, die am Rand gezackt sind. Ein Scheibenrührer ist eine rotierende Scheibe mit mehreren am Rand aufgesetzten Rührblättern und ein Schrägblattrührer weist mehrere am Rand bzw. an der Welle schräg aufgesetzte Rührblätter auf. Diese Rühr- oder Zerkleinerungsorgane sind in der Regel schnellrotierende Rührer, die hohe Scherfelder erzeugen können.

Wirbler sind quirl- oder rührerähnliche Mischwerkzeuge, die vielfältige geometrische Formen haben können und für eine gute Duchmischung und den Eintrag einer hohen Mischenergie sorgen.

Besonders bevorzugte Kombinationen von Reaktor- bzw. Mischertypen und Rühroder Zerkleinerungsorganen sind ausgewählt aus Schaufeltrockner mit zusätzlichen Messermühlen im unteren Bereich des Schaufeltrockners; List-Trockner, die sowohl einwellig (Diskotherm-Reaktoren) als auch zweiwellig sein können; Rührkessel mit Ankerrührer oder Wendelrührer und mindestens einer, bevorzugt 2 bis 4, besonders bevorzugt 3 bis 4 Dissolverscheiben oder mit Scheibenrührer, Schrägblattrührer, Turrax®-Rührer oder Ultraturrax® oder Messermühlen; Pflugscharmischer (Lödigemischer) mit eingebautem Zerhacker; Eirichmischer, in denen Wandanbackungen durch einen Wandabstreifer verhindert werden und die Scherfelder durch Wirbler aufgebracht werden.

Der Eirichmischer ist ein Intensivmischer, der dadurch charaktierisiert ist, daß er einen rotierenden Mischbehälter und ein wahlweise gleich- oder gegenläufig rotierendes Mischwerkzeug (Wirbler) besitzt. Das Mischwerkzeug kann eine sehr hohe Drehzahl von mehr als 2000 U/min erreichen. Bei den Mischwerkzeugen handelt es sich dabei um quirl- oder rührerähnliche Werkzeuge, die vielfältige geometrische Formen haben können und für eine gute Durchmischung und für den Eintrag einer hohen Mischenergie sorgen. Durch einen Wandschaber wird dabei verhindert, daß Material an der Wand anbäckt. Eirich-Intensivmischer sind von der Maschinenfabrik Gustav Eirich in Hardheim, Deutschland erhältlich.

Das Verfahren kann vorzugsweise auch in einem beheizbaren Vakuummischer durchgeführt werden. Vakuummischer gibt es z.B. von Eirich. Diese Mischer arbeiten nach dem sog. EVACTHERM®-Verfahren (von Eirich).

Anstelle eines in dem Reaktor angebrachten zweiten Rühr- oder Zerkleinerungsorgans kann in einer weiteren Ausführungsform der vorliegenden Erfindung ein externes Zerkleinerungsorgan für eine Naßzerkleinerung eingesetzt werden. Der Inhalt des Reaktors wird in dieser Ausführungsform über dieses Aggregat umgepumpt. Bevorzugte externe Zerkleinerungsorgane sind Naß-Rotor-Mühlen oder Zahnkranz-Dispergiermaschinen und Rührwerksmühlen.

Bei einer Herstellung von festen, kristallalkoholfreien Alkalimetallalkoholaten in einer einzigen Apparatur wird die Umsetzung des Alkalimetalls mit dem Alkohol bevorzugt bei einer Temperatur unterhalb der Schmelztemperatur des eingesetzten Alkalimetalls durchgeführt. Diese Ausführungsform des erfindungsgemäßen Verfahrens ist daher insbesondere zur Herstellung von festen, kristallalkoholfreien Alkalimetallalkoholaten geeignet, deren entsprechende Alkoholkomponente einen Siedepunkt unterhalb des Schmelzpunktes des eingesetzten Alkalimetalls aufweist. Ganz besonders bevorzugt ist daher die Herstellung von Na-tert.-butanolat aus tert.-Butanol (Siedepunkt: 82°C) und Natrium (Schmelzpunkt 98°C). Das Verfahren wird üblicherweise bei Normaldruck durchgeführt.

Zur Durchführung dieser Ausführungsform des erfindungsgemäßen Verfahrens wird der entsprechende Alkohol, bevorzugt tert.-Butanol, zusammen mit dem erfindungsgemäß eingesetzten Katalysator, bevorzugt Eisen(III)chlorid, in einem geeigneten Reaktor vorgelegt und geschmolzenes Alkalimetall, bevorzugt Natrium, über eine Düse oder ein Einleitrohr in die vorgelegte Reaktionsmischung bei Normaldruck eindosiert. Die Dosierung wird dabei vorzugsweise so durchgeführt, daß die Temperatur des Alkohols unterhalb der Schmelztemperatur des Alkalimetalls liegt. Durch die niedrige Temperatur erstarrt das eindosierte Alkalimetall zu Kügelchen und reagiert mit dem Alkohol zu dem entsprechenden Alkalimetallalkoholat ab. Die Rührorgane des Rührers sorgen dabei für eine intensive Durchmischung des Reaktionsgemisches. Die Durchmischung wird vorzugsweise durch den Einsatz eines zweiten Rühr- oder Zerkleinerungsorgans zusätzlich verbessert, wodurch eine weitere Erhöhung der Reaktionsgeschwindigkeit erzielt werden kann. Nach Beendigung der bei der Reaktion auftretenden Wasserstoffentwicklung wird der Reaktor auf Temperaturen von im allgemeinen 90 - 230°C, bevorzugt von 100 - 200°C, besonders bevorzugt von 110 - 180°C bei Normaldruck aufgeheizt. Bei Anlegen eines Vakuums von im allgemeinen bis max. 200 mbar, bevorzugt von 10 - 100 mbar, besonders bevorzugt von 10 - 30 mbar, können die angegebenen Temperaturbereiche unterschritten werden. Die Temperatur und das Vakuum sind so zu wählen, daß möglichst keine oder nur geringe Verluste durch Sublimation des Alkoholats auftreten. Der überschüssige Alkohol wird auf diese Weise entfernt und man erhält ein festes, kristallalkoholfreies Alkoholat in einer Reinheit von im allgemeinen >98 %, bevorzugt von >99 %. Dieses kann direkt in weiteren Reaktionen eingesetzt werden.

Wird die Herstellung von festen, kristallalkoholfreien Alkalimetallalkoholaten in einer einzigen Apparatur durchgeführt, so wird vorzugsweise eine Vorrichtung eingesetzt, umfassend:

a) einen heizbaren Reaktor A mit einem wandgängigen Rührorgan,

b) eine Einrichtung B zur Zugabe von flüssigem Alkalimetall in den Reaktor,

c) einen Kühler C zur Rückführung von während der Umsetzung verdampftem Alkohol in den Reaktor, über den während des im Anschluß an die Umsetzung erfolgenden Eindampfens und Trocknens abdestillierter überschüssiger Alkohol auskondensiert werden kann,

d) ein weiteres Rühr- oder Zerkleinerungsorgan D.

Entsprechende geeignete Reaktoren A, die zusätzlich eine Einrichtung zum Anlegen von Vakuum aufweisen können, sowie geeignete zweite Rühr- und Zerkleinerungsorgane wurden bereits vorstehend beschrieben.

Bei der Einrichtung B zur Zugabe von flüssigem Alkalimetall in den Reaktor handelt es sich bevorzugt um einen heizbaren Behälter, aus dem über ein Rohr, einen Hahn oder ein Rohr mit Hahn, z.B. einen heizbaren Tropftrichter, das flüssige Alkalimetall in den Reaktor dosiert wird. Die Dosierung kann beispielsweise mittels Schwerkraft, mittels Inertgasüberdruck oder mit einer Pumpe erfolgen.

Als Kühler C können praktisch alle Kühlertypen eingesetzt werden.

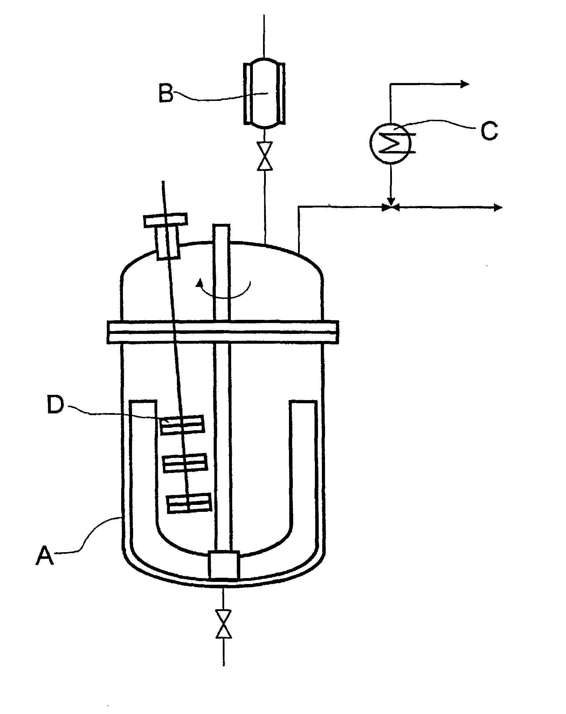

In Fig. 1 ist eine bevorzugte Ausführungsform einer geeigneten Vorrichtung schematisch dargestellt. Darin bedeuten:

A heizbarer Reaktor mit einem wandgängigen Rührorgan, am Beispiel eines Rührkessels mit Ankerrührer;

B Einrichtung zur Zugabe von flüssigem Alkalimetall;

C Kühler

D weiteres Rühr- oder Zerkleinerungsorgan, am Beispiel von 3 Dissolverscheiben.

Ein weiterer Gegenstand der vorliegenden Erfindung ist die Verwendung eines Übergangmetallsalzes als Katalysator, bevorzugt von Eisen(III)chlorid, bei der Herstellung von Alkalimetallalkoholaten aus Alkalimetallen und tertiären Alkoholen.

Dadurch kann die Umsetzung von Alkoholen, insbesondere von reaktionsträgen, tertiären Alkoholen mit niedrigen Siedepunkten, unter Normaldruck ohne zusätzliches hochsiedendes Lösungsmittel in kurzen Reaktionszeiten durchgeführt werden. Aufwendige Vorrichtungen zum Arbeiten unter Druck sind nicht notwendig.

Die nachfolgenden Beispiele erläutern die Erfindung zusätzlich.

Beispiel 1

4400 g tert.-Butanol wurden in einem 5 l Rührreaktor mit eingebauten Strömungsstörern, Rückflußkühler und Blattrührer (Drehzahl: 200 Upm) vorgelegt, auf Siedetemperatur erwärmt und mit 2 ml einer ca. 2 gew.-%igen Lösung von $FeCl_3*6H_2O$ in tert.-Butanol versetzt. Natrium (160 g) wurde im Stickstoffkasten blank geschnitten, in einen beheizten Doppelmanteltropftrichter gegeben und auf 120 - 130°C erwärmt, damit eine Schmelze entsteht. Innerhalb von 10 - 15 min. wurde das Natrium flüssig zum gerührten Ansatz zugetropft (Tropfendurchmesser ca. 1 - 5 mm). Es trat ein starkes Kochen am Rückfluß ein. Die Reaktion wurde anhand des freiwerdenden Wasserstoffs verfolgt. Nach ca. 4 h Laufzeit war keine Gasentwicklung mehr meßbar, und es hatte sich eine Suspension gebildet. Die Reaktion war beendet. Danach wurden Proben zur Titration entnommen (ca. 4 ml Suspension) und in Wasser eingewogen. Die Titration mit Salzsäure ergab eine Konzentration von 14,5 Gew.-% Natrium-tert.-Butanolat (Löslichkeit von Natriumtert.-butanolat in tert.-Butanol: < 10 %). Die entstandene Suspension wurde warm in einen 5-l-Schaufeltrockner überführt und bei einer Manteltemperatur von 150°C unter Normaldruck eingedampft und getrocknet. Es wurden 650 g festes Natriumtert.-butanolat mit einem Gehalt von > 99% erhalten.

Vergleichsbeispiel 2

Entsprechend dem obigen Beispiel wurden Natrium und tert.-Butanol miteinander umgesetzt, wobei aber auf die Zugabe des Katalysators verzichtet wurde. Nach der Zugabe des Natriums bildete sich ein großer Natriumklumpen. Nach ca. 6 - 8 h war eine dicke Suspension entstanden, die noch signifikante Mengen an Natriumkügelchen enthielt. Diese waren teilweise verkrustet und reagierten nur noch sehr langsam. Der Versuch wurde nach 10 h abgebrochen. Das Natrium war zu diesem Zeitpunkt noch nicht vollständig abreagiert.

Beispiel 3

Herstellung von Natrium-tert.-amylat

4400 g tert.-Amylalkohol wurden in einem 5-1-Rührgefäß mit eingebauten Strömungsstörern und einem Scheibenrührer vorgelegt, unter Rühren zum Sieden auf ca. 102°C erhitzt mit 2 ml einer ca. 2%igen Lösung von $FeCl_3*6H_2O$ in tert.-Butanol versetzt.

Natrium (300 g) wurde in einen beheizten Doppelmanteltropftrichter gegeben und auf 120 bis 130°C erwärmt, damit eine Schmelze entstand. Innerhalb von 45 Min. wurde das Natrium flüssig zum gerührten Ansatz gegeben (Rührerdrehzahl: 800 Upm). Natrium blieb bei dieser Temperatur flüssig und wurde mit dem Rührer dispergiert. Es trat ein starkes Kochen am Rückfluß ein. Die Reaktion wurde anhand des freiwerdenden Wasserstoffs verfolgt. Nach einer Nachreaktionszeit von 1 h war keine Gasentwicklung mehr meßbar. Die Reaktion war beendet. Die entstandene klare Lösung wurde über das Bodenventil warm in einen Rotationsverdampfer abgelassen und bei einer Badtemperatur von 130°C und Vakuum von ca. 30 mbar eingedampft und getrocknet.

Es wurden 1,4 kg festes Natrium-tert.-amylat erhalten mit einem Gehalt von > 99%. Die Konzentration an NaOH lag bei < 1%.

Vergleichsbeispiel 4

Die Umsetzung nach Beispiel 3 wurde wiederholt, wobei aber kein Katalysator zugegeben wurde. Die Umsetzung war in diesem Fall bei gleicher Drehzahl des Rührers erst nach 6 h beendet.

Beispiel 5

In einem 5 1-Rührreaktor, der mit einem wandgängigen, geschränkten Ankerrührer und drei Dissolverscheiben (auf einer Welle), Doppelmantel, Doppelmanteltropftrichter und Innenthermometer ausgerüstet war, wurden 5 1 tert.-Butanol vorgelegt und unter Rühren auf Siedetemperatur erwärmt (Kp: 82,5°C). Der Ankerrührer rotierte mit 90 Upm und die Dissolverscheiben mit 2000 Upm. Zu dem t-Butanol wurden 28 mg $FeCl_3$ zugefügt, Natrium (215 g) wurde im Stickstoffkasten blank geschnitten, in den Doppelmanteltropftrichter gegeben und durch Erwärmen auf 130°C unter Stickstoff aufgeschmolzen (F: 97,8°C). Das Natrium wurde innerhalb von 10 - 60 min flüssig zugetropft (Tropfendurchmesser ca. 1 - 10 mm). Der Reaktorinhalt wurde 6 - 8 h (bis die Wasserstoffentwicklung beendet war) bei Siedetemperatur gerührt. Anschließend wurde die Temperatur des Wärmeträgermediums erhöht und t-Butanol abdestilliert. Das Produkt wurde anschließend durch Erhöhung der Wandtemperatur auf 180°C getrocknet. Gegebenenfalls wurde die Trocknung durch Anlegen von Vakuum unterstützt. Es wurden 860 g Na-tert.-butanolat ausgetragen.

Alkoholatgehalt: > 98 %

NaOH-Gehalt: < 1,5 %

Restgehalt an nicht umgesetztem Natrium: < 10 ppm

Beispiel 6

In einem 5-1 Laborschaufeltrockner wurden 2414 g t-Butanol und 0,0176 g $FeCl_3$ vorgelegt und auf ca. 75 - 80°C erwärmt. 130 g Natrium wurden in einem doppelwandigen Tropftrichter aufgeschmolzen und anschließend flüssig in den Schaufeltrockner dosiert. Nach Reaktionsende (ca. 11 h) wurde die Manteltemperatur des Trockners langsam auf 180°C erhöht, überschüssiges t-Butanol abdestilliert und das Alkoholat getrocknet. Aus dem Schaufeltrockner wurden ca. 500 g Natrium-tert-butylat (Gehalt: > 99%) ausgetragen.

**Patentansprüche**

1.　Verfahren zur Herstellung von Alkalimetallalkoholaten durch Umsetzung von Alkalimetallen mit tertiären Alkoholen, **dadurch gekennzeichnet, daß** die Umsetzung in Gegenwart eines Übergangsmetallsalzes als Katalysator erfolgt.

2.　Verfahren nach Anspruch 1, **dadurch gekennzeichnet, daß** Eisen(III)chlorid als Katalysator eingesetzt wird.

3.　Verfahren nach Anspruch 1 oder 2, **dadurch gekennzeichnet, daß** das Übergangsmetallsalz, berechnet auf die eingesetzte Menge Alkalimetall, in einer Menge von 1 - 1000 ppm, berechnet als Metall, eingesetzt wird.

4.　Verfahren nach einem der Ansprüche 1 bis 3, **dadurch gekennzeichnet, daß** der Alkohol im Überschuß eingesetzt wird und gleichzeitig als Dispergiennedium und Lösungsmittel dient.

5.　Verfahren nach einem der Ansprüche 1 bis 4, **dadurch gekennzeichnet, daß** tert.-Butanol oder tert.-Amylalkohol eingesetzt werden.

6.　Verfahren nach einem der Ansprüche 1 bis 5, **dadurch gekennzeichnet, daß** die Umsetzung bei oder in der Nähe der Siedetemperatur des jeweiligen Alkohols durchgeführt wird.

7.　Verfahren nach einem der Ansprüche 1 bis 6, **dadurch gekennzeichnet, daß** als Alkalimetall Natrium eingesetzt wird.

8.　Verfahren nach einem der Ansprüche 1 bis 7, **dadurch gekennzeichnet, daß** die Umsetzung so durchgeführt wird, daß eine Suspension des Alkoholats in dem entsprechenden Alkohol entsteht.

9. Verfahren nach einem der Ansprüche 1 bis 8, **dadurch gekennzeichnet, daß** die Alkalimetallalkoholate im Anschluß an die Umsetzung durch gegebenenfalls Eindampfen von überschüssigem Alkohol und Trocknen in fester, kristallalkoholfreier Form gewonnen werden.

10. Verfahren nach Anspruch 9, **dadurch gekennzeichnet, daß** die Umsetzung, das Eindampfen und das Trocknen in einer einzigen Apparatur durchgeführt werden.

11. Verfahren nach Anspruch 10, **dadurch gekennzeichnet, daß** die Apparatur ein Reaktor mit einem wandgängigen Rührorgan ist.

12. Verfahren nach Anspruch 11, **dadurch gekennzeichnet, daß** als Reaktoren Reaktor- bzw. Mischertypen ausgewählt aus Schaufeltrockner, Diskotherm-Reaktor (List-Trockner), Rührkessel mit Ankerrührer, Rührkessel mit Wendelrührer, Pflugscharmischer (Lödigemischer) und Eirichmischer eingesetzt werden.

13. Verfahren nach Anspruch 12, **dadurch gekennzeichnet, daß** als Reaktor ein Schaufeltrockner oder ein Rührkessel mit Ankerrührer eingesetzt wird.

14. Verfahren nach einem der Ansprüche 11 bis 13, **dadurch gekennzeichnet, daß** die Apparatur zusätzlich zu dem Rührer ein weiteres Rühr- oder Zerkleinerungsorgan aufweist.

15. Verfahren nach Anspruch 14, **dadurch gekennzeichnet, daß** das weitere Rühroder Zerkleinerungsorgan ausgewählt aus Messermühlen, Zerhacker, Dissolverscheiben, Schreibenrührer, Schrägblattrührer, Turrax-Rührer oder Ultraturrax ist.

16. Verfahren nach Anspruch 15, **dadurch gekennzeichnet, daß** das weitere Rühroder Zerkleinerungsorgan ein externes Zerkleinerungsaggregat für eine Naßzerkleinerung ist

17. Verwendung eines Übergangsmetallsalzes als Katalysator bei der Herstellung von Alkalimetallalkoholaten aus Alkalimetallen und tertiären Alkoholen.

**Claims**

1. A process for preparing alkali metal alkoxides by reacting alkali metals with tertiary alcohols, wherein the reaction takes place in the presence of a transition metal salt as catalyst.

2. A process as claimed in claim 1, wherein iron(III) chloride is employed as catalyst.

3. A process as claimed in claim 1 or 2, wherein the transition metal salt is employed in an amount of 1 - 1000 ppm calculated as metal and based on the amount of alkali metal employed.

4. A process as claimed in one of claims 1 to 3, wherein the alcohol is employed in excess and serves simultaneously as dispersion medium and solvent.

5. A process as claimed in one of claims 1 to 4, wherein tert-butanol or tert-amyl alcohol is employed.

6. A process as claimed in one of claims 1 to 5, wherein the reaction is conducted at or close to the boiling point of the respective alcohol.

7. A process as claimed in one of claims 1 to 6, wherein sodium is employed as the alkali metal.

8. A process as claimed in one of claims 1 to 7, wherein the reaction is conducted so as to give a suspension of the alkoxide in the corresponding alcohol.

9. A process as claimed in one of claims 1 to 8, wherein the alkali metal alkoxides are obtained in solid form, free from alcohol of crystallization, following the reaction by optional evaporation of excess alcohol and drying.

10. A process as claimed in claim 9, wherein the reaction, evaporation, and drying are conducted in a single apparatus.

11. A process as claimed in claim 10, wherein the apparatus is a reactor having a close-clearance stirring element.

12. A process as claimed in claim 11, wherein reactors used comprise reactor types or mixer types selected from paddle dryers, Diskotherm reactors (List dryers), stirred vessels with anchor stirrer, stirred vessels with helical stirrer, plowshare mixers (Lödige mixers), and Eirich mixers.

13. A process as claimed in claim 12, wherein the reactor used is a paddle dryer or a stirred vessel with anchor stirrer.

14. A process as claimed in one of claims 11 to 13, wherein in addition to the stirrer the apparatus has a further stirring or comminuting element.

15. A process as claimed in claim 14, wherein the further stirring or comminuting element is selected from blade mills, choppers, dissolver disks, disk stirrers, inclined-blade stirrers, Turrax stirrers or Ultraturrax.

**16.** A process as claimed in claim 15, wherein the further stirring or comminuting element is an external comminuting unit for wet comminution.

**17.** The use of a transition metal salt as catalyst in the preparation of alkali metal alkoxides from alkali metals and tertiary alcohols.

**Revendications**

**1.** Procédé de production d'alcoolats de métaux alcalins par réaction de métaux alcalins avec des alcools tertiaires, **caractérisé en ce que** la réaction s'effectue en présence d'un sel de métal de transition comme catalyseur.

**2.** Procédé selon la revendication 1, **caractérisé en ce que** du chlorure ferrique est utilisé comme catalyseur.

**3.** Procédé selon la revendication 1 ou 2, **caractérisé en ce que** le sel de métal de transition, calculé par rapport à la quantité de métal alcalin mise en oeuvre, est mis en oeuvre dans une quantité de 1 à 1000 ppm, calculée à l'état de métal.

**4.** Procédé selon l'une des revendications 1 à 3, **caractérisé en ce que** l'alcool est mis en oeuvre en excédent et sert en même temps d'agent dispersant et de solvant.

**5.** Procédé selon l'une des revendications 1 à 4, **caractérisé en ce que** du tert.-butanol ou de l'alcool amylique tertiaire sont mis en oeuvre.

**6.** Procédé selon l'une des revendications 1 à 5, **caractérisé en ce que** la réaction est effectuée à ou à proximité de la température d'ébullition de l'alcool respectif.

**7.** Procédé selon l'une des revendications 1 à 6, **caractérisé en ce que** du sodium est utilisé comme métal alcalin.

**8.** Procédé selon l'une des revendications 1 à 7, **caractérisé en ce que** la réaction est effectuée de telle sorte qu'une suspension de l'alcoolat se forme dans l'alcool correspondant.

**9.** Procédé selon l'une des revendications 1 à 8, **caractérisé en ce que** les alcoolats de métaux alcalins sont obtenus à la suite de la réaction par évaporation éventuelle d'alcool excédentaire et séchage sous forme solide, exempte d'alcool cristallin.

**10.** Procédé selon l'une des revendications 9, **caractérisé en ce que** la réaction, l'évaporation et le séchage sont effectués dans un seul appareillage.

**11.** Procédé selon la revendication 10, **caractérisé en ce que** l'appareillage est un réacteur muni d'un organe d'agitation à parois mobiles.

**12.** Procédé selon la revendication 11, **caractérisé en ce que** les types de réacteurs ou de mélangeurs utilisés comme réacteurs sont choisis parmi les séchoirs à pales, les réacteurs Diskotherm (sécheurs List), les cuves malaxeuses à agitateur à ancre, cuves malaxeuses avec agitateur hélicoïdal, mélangeurs à soc de charrue (mélangeur Lödige) et mélangeurs Eirich.

**13.** Procédé selon la revendication 12, **caractérisé en ce qu'**un sécheur à pales ou une cuve malaxeuse à agitateur à ancre sont utilisés comme réacteur.

**14.** Procédé selon l'une des revendications 11 à 13, **caractérisé en ce que** l'appareillage comporte un organe d'agitation ou de broyage supplémentaire en plus de l'agitateur.

**15.** Procédé selon la revendication 14, **caractérisé en ce que** l'organe d'agitation ou de broyage supplémentaire est choisi parmi les broyeurs à lames, hacheurs, disques dissolveurs, agitateurs à disques, agitateurs à lames obliques, agitateurs Turrax ou Ultraturrax.

**16.** Procédé selon la revendication 15, **caractérisé en ce que** l'organe d'agitation ou de broyage supplémentaire est un groupe de broyage externe pour un broyage mouillé.

**17.** Utilisation d'un sel de métal de transition comme catalyseur pour la production d'alcoolats de métaux alcalins à partir de métaux alcalins et alcools tertiaires.